# EUROPEAN PATENT APPLICATION

(11) **EP 0 908 192 A2**
(43) Date of publication of application: **14.04.1999**
(21) Application number: 98304807.5
(22) Date of filing: 17.06.1998
(51) Int. Cl.: A61M 5/32

(54) **Safety syringe for intravenous injection with a guided plunger**

(30) Priority: 16.09.1997 CN 97116904
(71) Applicant: Chen, Long-Hsiung, Taipei (TW)
(72) Inventor: Chen, Long-Hsiung, Taipei (TW)
(74) Representative: Selby-Lowndes, Guy Francis Charles

(57) **Abstract**

The present invention relates to a safety syringe for intravenous injection with a guided plunger, which comprises a barrel, a needle unit, and a plunger. An outlet is eccentrically disposed on the top of the barrel. The needle unit includes a needle and a spindle shaped needle holder mounted inside the outlet. A connecting stem has a arrowheaded mortise disposed under the spindle shaped needle holder. A plunger has a plunger head on the top with a arrowheaded tenon eccentrically disposed thereon. A supporting plate disposed under the plunger head having two guide notches inside the barrel is matched with two longitudinal guide strips that the arrowheaded tenon can exactly toward the needle unit. After finished the injection, the arrowhead beetle and the needle unit are connected together, the needle unit is pulled to the side of the barrel and deformed.

## Description

### FIELD OF THE INVENTION

The present invention relates to a safety syringe for intravenous injection with a guided plunger, more particularly to a syringe which the needle and the needle holder can be pulled into the barrel after use to prevent accidental needle stick.

### BACKGROUND OF THE INVENTION

In the past, needle stick injuries suffered by medical personal and others in the course of using intravenous syringe have present a serious problem. Serious problem such as hepatitis and AIDS may be transmitted by needle stick injuries, resulting in the needless suffering, and possibly even in the death. Health care workers may have accidental needle stick injuries while curing for AIDS or hepatitis patients. Avoiding accidental needles stick is very important for health care workers.

The needle of a safety syringe is normally provided in a central portion of the syringe, which suitable for hypodermic or intramuscular injection. However, if such a syringe is not suitable used for intravenous injection of larger quantity of liquid medicine filled in large size syringe. The needle should be eccentricity formed from the center line of the syringe for a ergonomics injection. Therefore, an eccentrically-positioned needle of syringe for a ergonomics injection is better than a centrically-position needle of syringe.

### SUMMARY OF THE INVENTION

It is an object of the present invention to provide a safety syringe for intervenes injection with a guided plunge, wherein the bottom of the hooked under the needle holder is formed rank surface so that the needle can be inclined after pulling to the side of the barrel, and wherein two beetles inside the barrel toward two notches on the flange so that the plunger can not be turned and exactly toward the needle unit.

### BRIEF DESCRIPTIONS OF THE DRAWINGS

The present invention can be better understood by detailed descriptions of the follow drawings, in which ;

Fig. 1 is an illustration showing the present invention in using.

Fig. 2 is an illustration showing the present invention when finishing the injection.

Fig. 2A is a section view of the bottom of the needle holder of Fig. 2.

Fig 3 is a sectional view of the present invention when viewed from A-A direction of Fig. 1.

Fig 4 is a sectional view of the present invention when viewed from B-B direction of Fig. 1.

Fig 5 shows the present invention having a needle retracted in syringe after finished the injection.

Fig. 5A is a sectional view of the bottom of the needle holder of Fig. 5.

Fig. 6 shows a blocking effect for retarding an outwardly protruding needle in accordance with the present invention.

Fig. 6A is an illustration showing the present invetion when the plunger is pushed forwardly and the needle is damaged and enclosed inside the barrel.

### DETAILED DESCRIPTIONS OF THE INVENTION

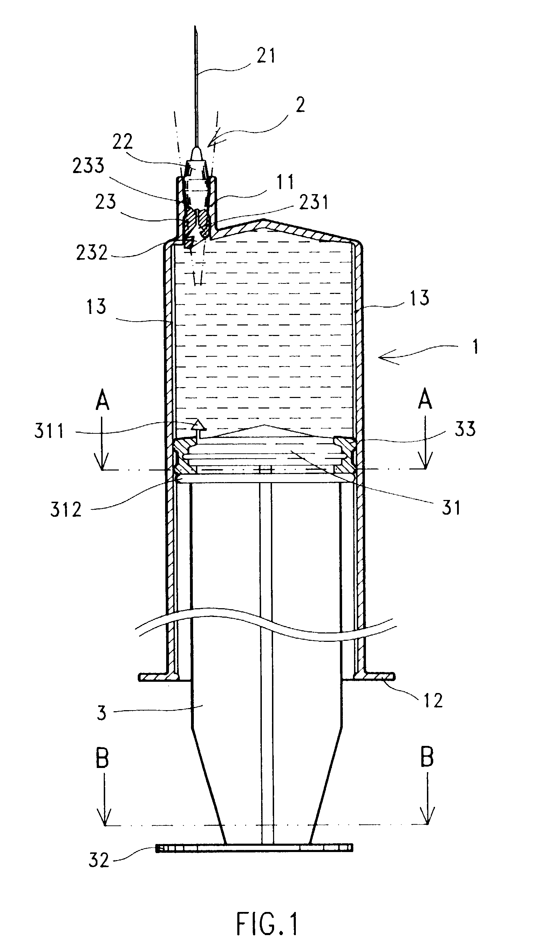

Please refer to Fig. 1, which shows the present invention in using. As shown in Fig. 1, the present invention includes a barrel 1, a needle unit 2, and the plunger 3. The needle unit 2 having a cannula needle 21, a spindle-shaped needle holder 22 held the rear portion of the cannula needle 21 therein, a connecting stem 23 extended downwardly from the needle holder 22 having an inclined bottom surface 230 thereat, 1 an inclined arrowheaded mortise 231 disposed in the connecting stem 23 having a downward flare opening 232 at the inclined bottom surface 230 and an upward through hole 233 extended from a tip end of the arrowheaded mortise 231 for leading the medical liquid into the cannula needle 21. The barrel 1 having a reduced tubular outlet 11 disposed on a conical front portion thereof for mounting the needle holder 22 thereinto, an outward flange 12 disposed at a bottom edge of an opened rear end thereof, two longitudinal guide strips 13 unsymmetrically disposed to the opposite side of the inner surface thereof. The plunger 3 comprising a plunger head 31 having an arrowheaded tenon 311 eccentrically disposed on a conical top surface thereof, a supporting plate 312 disposed under the plunger head 31 having two guide notches 313 engaged with the guide strips 13, a "+" sectional four-blade plunger rod 310 extended from the plunger head 31 to an end plate 32 and a sealing ring 33 inlied into the periphery of the plunger head 31 and supported by the supporting plate 312.

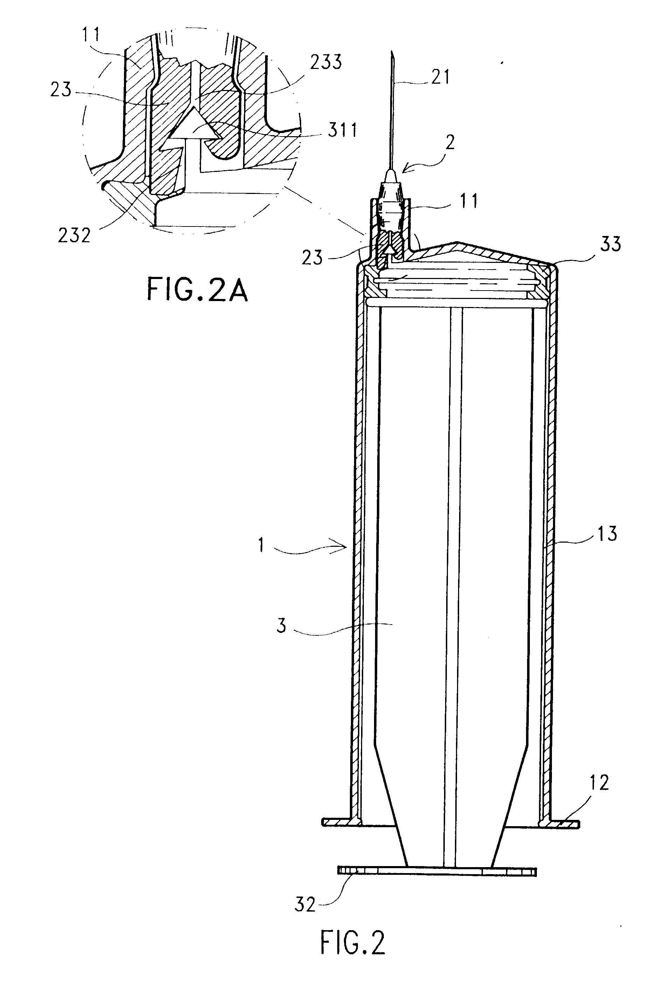

Please refer to Fig. 2, which shows the present invention when finishing the injection. The plunger 3 is pushed forward so that the arrowheaded tenon 311 is connected with the arrowheaded mortise 231 of the connecting stem 23.

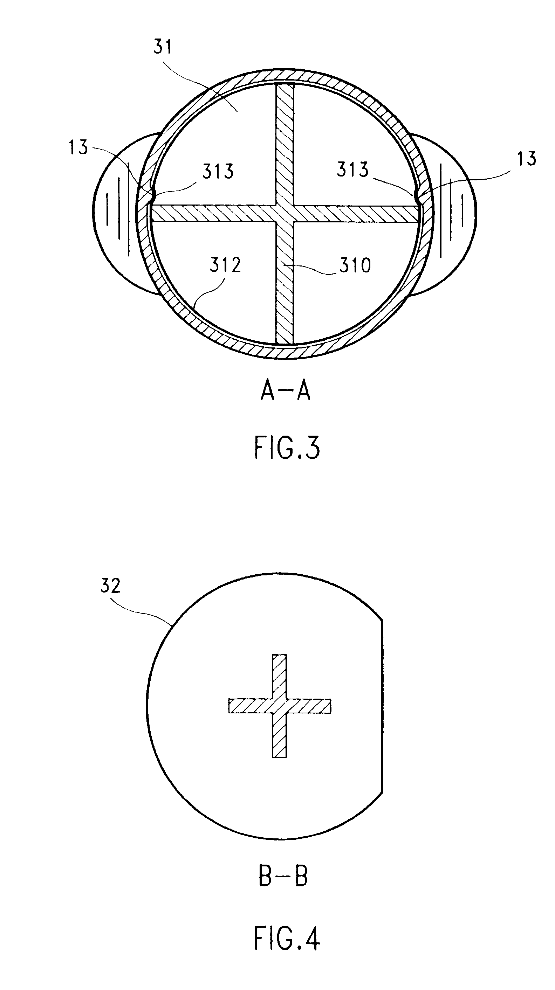

Please refer to Fig. 3, wherein two guide notches 313 of the supporting plate 312 of the plunger 3 are engaged with the guide strips 13 inside the barrel when the plunger 3 is pushed forwardly by finger, the plunger 3 can not be turned so as to keep the direction that the arrowheaded 311 be forced into the mortise 231 through the flange opening 232 of the connecting stem 23.

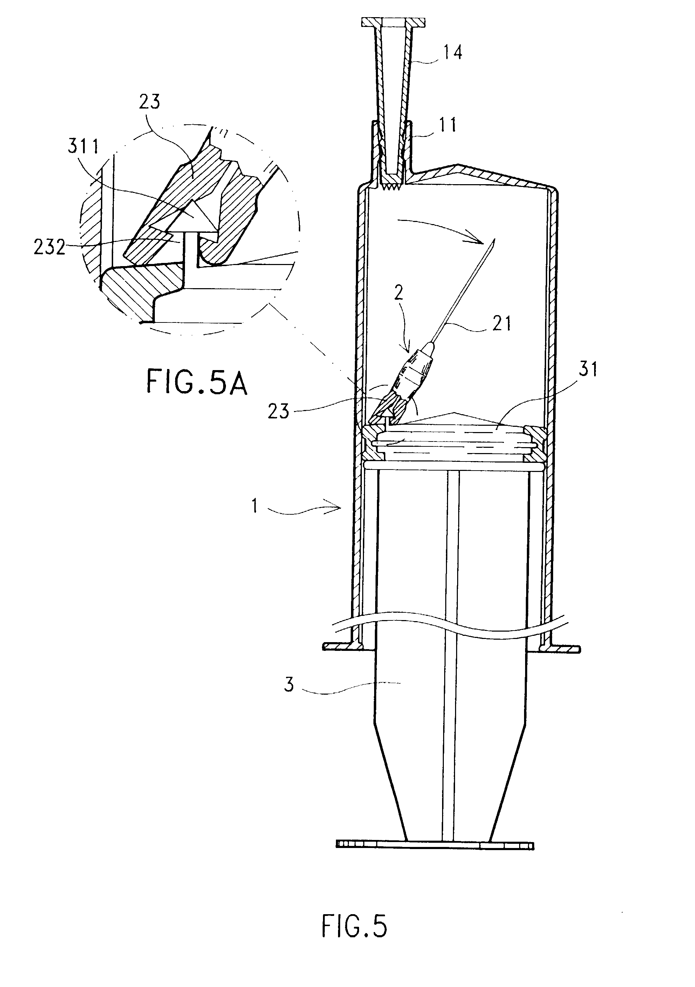

Please refer to Fig. 5, when the plunger 3 is pulled backward and the needle unit 3 is bringing into the barrel 1, the inclined bottom surface of the connecting stem 23, the inclined arrowheaded mortise 231, and the conical surface of the plunger head 31 make the needle 21 to incline to leave the reduced tubular outlet 11 far away. According to many experiments, it makes the needle 21 to incline mainly because of the inclined bottom.

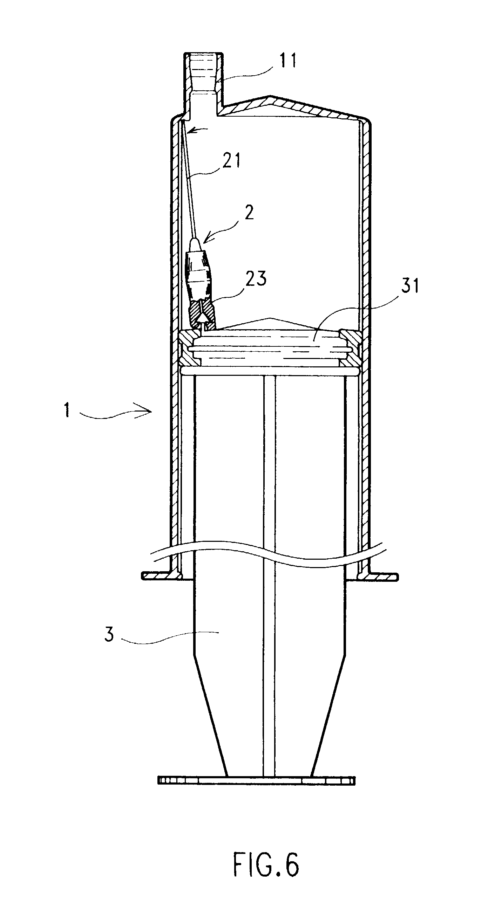

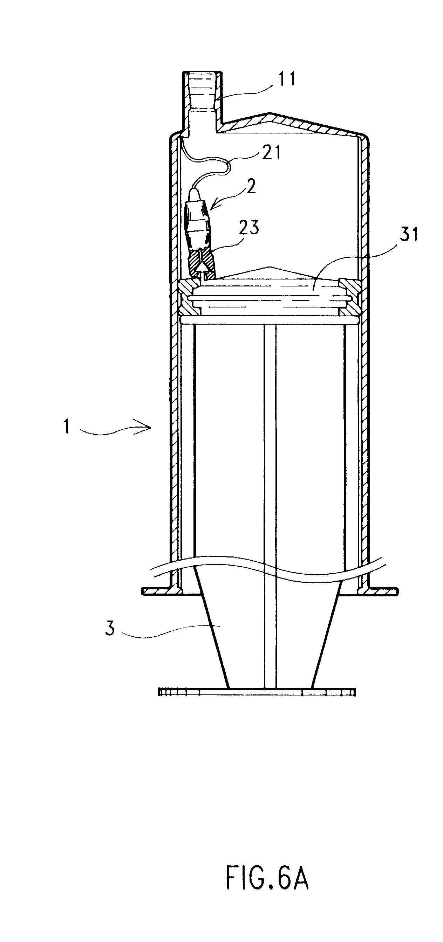

Please refer to Fig. 6, wherein the plunger 3 is pushed forward again after the plunger and the needle 21 is damaged while the needle 21 can not be forced out from the barrel 1. A needle cap 14 is reversely inserted into the outlet 11 of the barrel 1.

The above embodiments can be modified by any skillful person in the art without departing the spirit and scope of the accompanying claims.

## Claims

1. A safety syringe for intervenes injection with a guided plunger, comprising :
a barrel having a reduced tubular outlet eccentrically disposed on a conical front portion an outward flange to a bottom edge of a rear portion,
a needle unit having a cannula needle fixed in a spindle shaped needle holder mounted in said outlet of said barrel, a connecting stem extended downwardly from a rear end of said needle holder having an inclined bottom surface and a conical arrowheaded mortise disposed inside said connecting stem with an upward through hole and a downward flaring opening,
a plunger having a plunger head, a arrowheaded tenon eccentrically disposed on said plunger head and toward said mortise of said connecting stem, a sealing ring inlied around said plunger head, a supporting plate disposed under said plunger head for supporting said sealing ring in place having two guide notches matched with said guide strips in said barrel.

2. The safety syringe for intravenous injection with connecting stem the hooked is inclined.

3. The safety syringe for intravenous injection with a guided plunger according to claims, wherein a needle cap is reversely inserted said outlet of said barrel so that said needle can be damaged and enclosed inside said barrel.
